# EUROPEAN PATENT APPLICATION

(11) **EP 2 072 518 A1**
(43) Date of publication of application: **24.06.2009**
(21) Application number: 08172571.5
(22) Date of filing: 22.12.2008
(51) Int. Cl.: C07D 487/04, A61K 31/519, A61P 35/00

(54) **Stable Amorphous Form of Pemextred Disodium**

(30) Priority: 23.12.2007 IN ME23132007
(71) Applicant: Sun Pharma Advanced Research Company Limited, Andheri (East) Mumbai 400 093 (IN)
(72) Inventor: Patel, Nileshkumar Sureshbhai, Baroda-390 020, Gujarat (IN); Kilaru,Srinivasu, Baroda-390 020, Gujarat (IN); Thennati, Rajamannar, Baroda-390 020, Gujarat (IN)
(74) Representative: Atkinson, Jonathan David Mark

(57) **Abstract**

A stable amorphous form of pemetrexed disodium of formula I 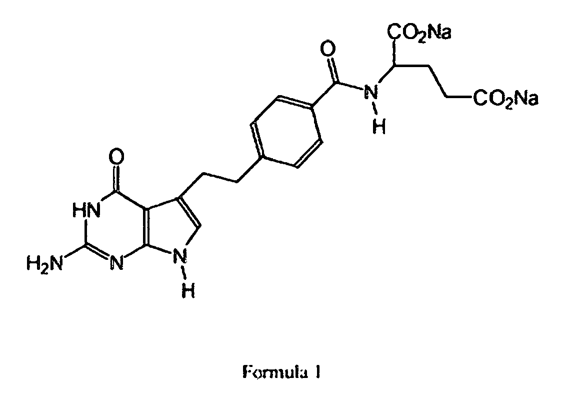 Amorphous pemetrexed disodium characterized by peaks in Raman spectrum at about (± 2cm⁻¹) 3064, 2921, 1611, 1534, 1438, 1343, 1293, 1190, 1157, 1076, 1010, 903, 872, 820, 639, 533, 373, 103 cm⁻¹.

## Description

### FIELD OF THE INVENTION

The present invention relates to stable amorphous form of pemetrexed disodium of formula 1 -

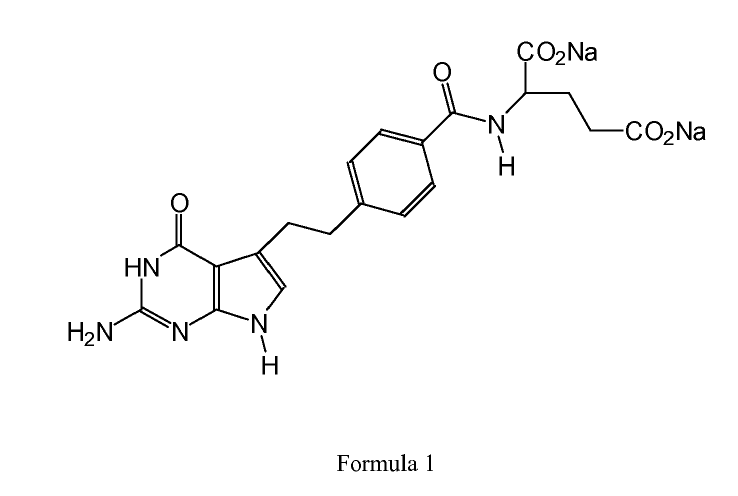

### BACKGROUND OF THE INVENTION

Pemetrexed disodium is a multitargeted antifolate agent approved as a single agent for the treatment of non-small cell lung cancer, and in combination with cisplatin for the treatment of patient with malignant pleural mesothelioma, under the trade name Alimta^{®}.
Pemetrexed disodium is available in a number of crystalline forms.

Barnett et al, Organic Process Research & Development, 1999, 3, 184-188 discloses synthesis and crystallization of pemetrexed disodium from water-ethanol. The product obtained by the process disclosed herein is the 2.5 hydrate of pemetrexed disodium.

United States patent number 7,138,521 discloses a crystalline heptahydrate form of pemetrexed disodium, which has enhanced stability when compared to the known 2.5 hydrate.

To date workers have concentrated on producing stable crystalline forms of pemetrexed disodium and there has been no disclosure of any non-crystalline form of this active.

We have now found a new form of pemetrexed disodium, which is an amorphous form, as characterized by powder X-ray diffraction. Surprisingly, we have found that it is possible to prepare an amorphous form of pemetrexed disodium and that this form is stable. The amorphous form of the invention is stable contrary to expectations. The amorphous form of pemetrexed disodium of the present invention is stable as it retains it's amorphous character under a variety of storage conditions. The amorphous form of the present invention is particularly advantageously characterized by a bulk density in the range of 0.15 to 0.35 gm/ml.

### SUMMARY OF THE INVENTION

The present invention provides a stable amorphous form of pemetrexed disodium.

The present invention provides a process for preparing the amorphous form of pemetrexed disodium comprising dissolving pemetrexed disodium in a solvent, to obtain a solution, optionally filtering the solution and recovering the amorphous form from the solution.

The present invention provides a pharmaceutical composition comprising the amorphous form of pemetrexed disodium and pharmaceutically acceptable excipients.

### DETAILED DESCRIPTION OF THE INVENTION

The present invention provides a stable amorphous form of pemetrexed disodium.

In one embodiment the amorphous pemetrexed disodium is characterized by the X-ray diffraction pattern of Figure 1.

In another embodiment, the amorphous pemetrexed disodium is characterized by differential scanning calorimetry ((DSC) profile (Figure 2).

In another embodiment, the amorphous pemetrexed disodium is characterized by peaks in the Raman spectrum at about 3064, 2921, 1611, 1534, 1438, 1343, 1293, 1190, 1157, 1076, 1010, 903, 872, 820, 639, 533, 373, 103 cm⁻¹. The tolerance of the measurements is ± 2 cm⁻¹. Table 1 in Example 1 also depicts the Raman spectra of amorphous form of the present invention.

In another embodiment, the amorphous pemetrexed disodium is characterized by differential scanning calorimetry (DSC) in which it exhibits an endothermic thermal event at about 244°C and more specifically at 243.76°C. The tolerance of the measurement is ± 1°C. There is an exothermic thermal event at about 292°C, and more specifically at about 291.94°C.

In a further embodiment the amorphous form of pemetrexed disodium is also characterized by the Raman Spectra substantially shown in Figure 3.

The amorphous pemetrexed can be characterized by any of the above mentioned methods individually, or in any combination of these individual methods.

In one embodiment the amorphous form of pemetrexed disodium of the present invention has a water content ranging from about 5% to about 21%.

In one embodiment the amorphous form of pemetrexed disodium of the present invention is stable, as it retains its amorphous nature and lacks significant impurity formation when stored under varying temperature and humidity conditions.

The expression "stable" when used in conjunction with amorphous pemetrexed disodium means that, within the scope of sound pharmacological judgment, the material can be stored without significant chemical degradation or transformation of amorphous form. The expression includes pemetrexed disodium that can be stored under a suitable atmosphere without transformation, or without significant transformation, of its amorphous form and without more than 5% degradation over a period of at least 28 days, and more preferably over a period of at least 3 months, and most preferably over a period of at least 6 months. Preferably, the degradation is less than 2%, and more preferably less than 1%. Percentage degradation may be determined by HPLC as described herein. In embodiments, the suitable atmosphere is an inert gas, e.g. nitrogen. In embodiments, the storage temperature is not more than 40°C, e.g. not more than 25°C. Included are embodiments in which the storage conditions include relative humidity not exceeding 80%, e.g. not exceeding 60%. One suitable storage condition is 25°C/60% relative humidity.

The term "stable", as used herein, thus implies that the pemetrexed disodium of the present invention maintains its amorphous form and lacks significant formation of impurities, while being stored as described herein.

In one embodiment the amorphous form of pemetrexed disodium of the present invention remains stable when subjected to the following real time and accelerated conditions wherein the amorphous form of pemetrexed disodium was stored
● at 2-8°C for a period of six months
● 25°C/60 % relative humidity for a period of six months
in that the amorphous form is retained as indicated by XRD and there was no significant formation of impurities as measured by HPLC when the samples were analysed after a period of six months.

The samples of amorphous form of pemetrexed disodium were stored in double seal poly bags followed by aluminum bag sealed under nitrogen with oxygen trapper.

In one embodiment the amorphous form of pemetrexed disodium (XRD as depicted in Figure 4) has an initial moisture content of about 9.0 %, and HPLC purity of 99.68% as determined by standard analytical procedures known in the art. In another embodiment the amorphous form of pemetrexed disodium (XRD as depicted in Figure 5) has an initial moisture content of about 16.0 %, and HPLC purity of 99.66% as determined by standard analytical procedures known in the art. Example 5 discloses that the above amorphous form of pemetrexed disodium of the present invention is stable when stored under accelerated conditions as it does not transform to any other form as indicated by the powder X-ray diffractograms (XRD) (figures 6-10) which clearly indicate that the amorphous form is conserved and there is no significant formation of impurity as indicated by the HPLC purity values after storing under these conditions for a period of six months

Examples 5 and 6 also disclose the moisture content variation in the samples of amorphous pemetrexed disodium of the present invention upon storage under various conditions Thus depending on the storage conditions, the packaging material and the initial moisture content, the moisture content in the samples on storage may vary from 5-21%.; however under all circumstances the amorphous form was conserved and there was no significant formation of impurities as indicated by HPLC values despite varying the moisture content indicating that the amorphous form of pemetrexed disodium of the present invention is stable.

In one embodiment the amorphous form of pemetrexed disodium of the present invention exhibits bulk density in the range of 0.15 to 0.35gm/ml.

In one embodiment the amorphous form of pemetrexed disodium of the present invention exhibits tapped density in the range of 0.25 to 0.45 gm//ml.

As referred to herein the term "bulk density" is the weight of the sample divided by its non packed volume and the term "tapped density" is the weight of the sample divided by its packed volume. The units of bulk density are grams (gm) per cubic centimeter (cc) or grams (gm) per milliliter (ml). A powder having low bulk density will be lightweight, fluffy and have greater surface area. A powder with high density will be much more compact and dense, exist as harder particles and will result in a more flowable product compared to powder with low bulk density.

In a preferred embodiment the amorphous form of pemetrexed disodium of the present invention exhibits a bulk density in the range of 0.21 to 0.24 gm/ml, and preferably a bulk density of 0.226 gm/ml ± 0.01 gm/ml.

In another preferred embodiment the amorphous form of pemetrexed disodium of the present invention exhibits a tapped density in the range 0.35 to 0.39 gm/ml, and preferably a bulk density of 0.369 gm/ml ± 0.01 gm/ml.

The present invention also relates to a process for the preparation of an amorphous form of pemetrexed disodium.

In one embodiment the present invention provides a process for preparing the amorphous form of pemetrexed disodium comprising the steps of
a) dissolving pemetrexed disodium in a solvent to obtain a solution;
b) optionally filtering the solution ; and
c) recovering the amorphous form of pemetrexed disodium from the solution.

The term "solvent", as used herein, includes any solvent or a solvent mixture in which pemetrexed disodium is soluble. The solvent may be selected from the group consisting of water, and a mixture water and alkanols selected preferably C₁₋₁₀ alkanols, from methanol, ethanol, isopropanol and the like.

Thus in one embodiment the solvent is water. In an alternative embodiment the solvent is a mixture of water and one or more alkanols.

If required the solution of pemetrexed disodium in solvent is filtered.

The amorphous form of pemetrexed disodium of the present invention may then be obtained by drying the solution of pemetrexed disodium in solvent. Conventional processes such as freeze drying, spray drying, flash drying and the like may be used for recovering the amorphous form pemetrexed disodium from the solution of pemetrexed disodium. Preferably the amorphous form is a freeze-dried or a spray-dried product. Most preferably, it is a freeze-dried product.

In one preferred embodiment the solvent for dissolving pemetrexed disodium is water.

In one embodiment of the present invention, pemetrexed disodium is dissolved in water to obtain a clear solution. This solution is then freeze dried to obtain the amorphous form of the pemetrexed disodium.

In one embodiment about 5 to 15 volumes of water are used for dissolving the pemetrexed disodium. In a preferred embodiment about 8 to 12 volumes of water are used.

In one embodiment the pH of the pemetrexed disodium solution in water prior to freeze drying is in the range of 6.5 to 7.5.In a preferred embodiment the pH of the pemetrexed disodium solution in water prior to freeze drying is about 6.95.

The freeze drying is carried out at the temperature in the range of -40 to +25°C and vacuum in the range of 0.1mm to 10mm. The freeze drying is carried out for a period of 15 to 60 hours. Based on the time of freeze drying and batch size, the water content of the amorphous form of the present invention can be varied in the range of 5-21%.

In one instance when the freeze drying is carried out for 28 hours an amorphous form of pemetrexed disodium with a water content of 7.46% is obtained. In another instance when the freeze drying is carried out for 53 hours an amorphous form of pemetrexed disodium with a water content of 5.82% is obtained. For larger batch sizes depending on the desired water content, the freeze drying time may be increased.

The pemetrexed disodium used for preparing the amorphous form of pemetrexed disodium of the instant invention may be prepared by any process known in the art. Preferably the starting pemetrexed disodium is prepared by dissolving the pemetrexed diacid in a solution of sodium hydroxide and water. The pemetrexed disodium is then precipitated by adding ethanol. The precipitated pemetrexed disodium is then isolated and used for preparing the amorphous form.

The amorphous pemetrexed disodium can be administered alone but will generally be administered in admixture with a pharmaceutically acceptable diluent or carrier selected with regard to the intended route of administration and standard pharmaceutical practice.
The amorphous form of pemetrexed disodium of the present invention may be formulated into conventional dosage forms such as, for example, tablets, pills, suspensions, emulsions, granules, capsules, injection preparations suitable for reconstitution and the like.

For example, it may be administered orally in the form of tablets containing such excipients as starch or lactose, or in capsules either alone or in admixture with excipients, or in the form of elixirs or suspensions containing flavouring or colouring agents. It may be injected parenterally, for example, intravenously, intramuscularly or subcutaneously.

For parenteral administration, it is best used in the form of a sterile aqueous solution which may contain other solutes, for example, enough salts or glucose to make the solution isotonic with blood.

The amorphous pemetrexed disodium may be administered as capsule formulations. Such formulations may be prepared by mixing the amorphous material together with suitable carriers or excipients such as microcrystalline cellulose, dried maize starch, colloidal silicon dioxide and magnesium stearate.

The amorphous form of pemetrexed disodium of the present invention may be used alone or in combination with one or more other therapeutically active agents.

It will be appreciated that reference to medical treatment means curative, palliative or prophylactic treatment.

The present invention also relates to the use of amorphous pemetrexed disodium for treating cancer, and in particular for treating non-small cell lung cancer as described below.

In particular, the amorphous form can be used, under the supervision of qualified professionals, to inhibit the growth of neoplasms including choriocarcinoma, leukemia, adenocarcinoma of the female breast, epidermid cancers of the head and neck, squamous or small-cell lung cancer, and various lymphosarcomas. It can also be used to treat mycosis fungoides and psoriasis.

The compounds preferably are administered parenterally, alone or in combination with other therapeutic agents including other anti-neoplastic agents, steroids, etc., to a mammal in need of medical treatment. Parenteral routes of administration include intramuscular, intrathecal, intravenous and intra-arterial. Dosage regimens must be titrated to the particular neoplasm, the condition of the patient, and the response but generally doses will be from about 10 to about 100 mg/day for 5-10 days or single daily administration of 250-500 mg, repeated periodically; e.g. every 14 days. The term mammal includes a human patient.

The present invention is further illustrated by the following examples which are provided merely to be exemplary of the invention and are not intended to limit the scope of the invention.

### Examples

### Experimental Methods

### General description of the equipment.

X-ray diffraction data were acquired using a PANalyticalX'pert PRO X-ray diffractometer model.

System description: K_{α1}=1.54060 A⁰, voltage 45 kV, current 40 mA, Xray source : Cu..

Experiment parameters: pattern measured between 2θ=4° and 2θ=40° with 0.05° increments; count time was 0.5 second per increment.

DSC was performed using Metler Toledo DSC822^{e}.

Freeze drying was performed on a freeze dryer by Martinchrist alpha 1-4 LD plus model.

Bulk and tapped densities were measured using Electrolab ETD-1020 tap density tester (USP).

The water content measurements were carried out using a METTLER TOLEDO Model: DL31Kar1 Fischer Titrator according to standard procedures.

Purity by HPLC was performed by Waters 2690 Seperations module.system.

Raman spectra was recorded on - FT-Raman spectrophotometer ,Make-Bruker
Optics,Switzerland,Model-RFS 100/S
Laser- 750 mW Nd: YAG laser operating at 1064nm.
Detector-Liquid nitrogen cooled Germanium detector.
No. of scans-50 (Scanning ranges from 3500cm-1 to -999cm-1)
Laser power-350mW.

### Example 1

### Table 1

| **Raman spectra of 4 batches of amotrphous pemetrexed of the present invention** | | | |
|---|---|---|---|
| | | | |
| 3066 | 3066 | 3063 | 3065 |
| 2925 | 2919 | 2921 | 2920 |
| 1611 | 1611 | 1611 | 1611 |
| 1535 | 1534 | 1534 | 1534 |
| 1438 | 1438 | 1438 | 1438 |
| 1348 | 1343 | 1343 | 1343 |
| 1292 | 1294 | 1292 | 1292 |
| 1189 | 1191 | 1189 | 1191 |
| 1155 | 1158 | 1156 | 1157 |
| 1078 | 1075 | 1076 | 1076 |
| 1008 | 1011 | 1009 | 1012 |
| 905 | 903 | 902 | 904 |
| 872 | 872 | 873 | 871 |
| 821 | 821 | 820 | 820 |
| 639 | 640 | 639 | 639 |
| 534 | 534 | 532 | 533 |
| 367 | 371 | 372 | 375 |
| 104 | 103 | 103 | 103 |

### Example 2

5.0 gm Pemetrexed disodium is dissolved in 50 ml demineralised water at 40-45⁰C and filtered to obtain a particle-free filtrate. The filtrate is then freeze dried at about -20⁰C to 0⁰C, at about 5 mm vacuum, using CHRiST freeze-drier for 28 hours to obtain 5.25 g of amorphous pemetrexed disodium.
X-ray diffraction data as indicated in figure 1.
Water content: 7.46%
HPLC purity 99.66%
Bulk density: 0.226gm/ml
Tapped density: 0.369gm/ml

### Example 3

13.2 gm Pemetrexed disodium was dissolved in 80 ml demineralised water at 40-45⁰C. 20 ml of additional demineralised water was added and the solution is then freeze dried at about -20 to 0⁰C, at about 1-2 mm vacuum, using CHRiST freeze-drier for 20 hours to obtain 10.5 g of amorphous pemetrexed disodium with an initial moisture content of 11.38% to 12.02%.

This material was dried under high vacuum till moisture content was 9% .
X-ray diffraction data as indicated in figure 4indicates amorphous form is retained.
Water content: 9%
HPLC purity 99.68%

The material was kept outside at room temperature for 15 hrs.
X-ray diffraction data as indicated in figure 5 indicates amorphous form is retained.
Water content: 16%
HPLC purity 99.66%

### Example 4

Solution of 0.074 Kg of Sodium hydroxide in 0.4 L Water for Injection is added to the suspension of 0.4 Kg Pemetrexed diacid in 1.2 L Water for Injection below 25°C. Obtained slight suspension is heated at 40-45°C and filtered. 6.0 L of Abs. Ethanol is added to the filtrate below 30°C and resulting precipitated product is stirred for 1.0 hr, filtered, washed with 0.8 L Abs. Ethanol and suck dried for 30-60 min. Above isolated wet material is dissolved in 3.2 L Water for Injection at 40°C, filtered and washed with 0.8 L Water for Injection. Resulting solution is freeze dried in Virtis freeze drier , Model No. : Genius 25XL, Sr No. : 214706 for 87 hrs (12 hrs for freezing + 76 hrs for drying) at - 40°C to 0° C using vacuum.
Yield = 398 gm
water content = 6.82%
HPLC purity = 99.61%

### Example 5

### Amorphous Pemetrexed disodium stability study

This example demonstrates the stability of amorphous form of pemetrexed obtained in example 2 with initial moisture content of 9 % and 16 %.

The samples were packed in double seal poly bags followed by Aluminium bag sealed under nitrogen with oxygen trapper and stored
● at 2-8°C for a period of six months
● 25°C/60 % relative humidity (RH) for a period of six months.
The purity of the sample was checked by HPLC and XRD was recorded for the samples stored under these conditions after 2 months and 6 months.

### I. Amorphous form of pemetrexed disodium, with moisture content of 9% prior to storage

### (a) Condition: 25°C RH 60%

| | HPLC | % Moisture Content | XRD |
|---|---|---|---|
| Initial | 99.68 | 9.04% | Amorphous |
| 2 Months | 99.72 | 8.39% | |
| 6 Months | 99.67 | 8.85% | Amorphous |
| | | | (figure 6) |

### (b) Condition: 2-8°C

| | HPLC | % Moisture Content | XRD |
|---|---|---|---|
| Initial | 99.68 | 9.04% | Amorphous |
| 2 Months | 99.72 | 8.96% | |
| 6 Months | 99.62 | 9.39% | Amorphous |
| | | | (figure 7) |

### II. Amorphous form of pemetrexed disodium, with moisture content of 16% prior to storage

### a. Condition: 25°C RH 60%

| | HPLC | % MC | XRD |
|---|---|---|---|
| Initial | 99.66 | 15.92% | Amorphous |
| 2 Months | 99.72 | 10.26% | |
| 6 Months | 99.63 | 11.45% | Amorphous |
| | | | (Figure 8) |

### b. Condition: 2-8°C

| | HPLC | % MC | XRD |
|---|---|---|---|
| Initial | 99.66 | 15.92% | Amorphous |
| 2 Months | 99.72 | 11.68% | |
| 6 Months | 99.59 | 12.31% | Amorphous |
| | | | (Figure 9) |

The above results indicate that all the samples tested conserved the amorphous form after being stored at the specified storage period under specific conditions. Further more there was no significant change in the initial chemical purity after storage.

### Example 6

This example demonstrates that amorphous form is conserved under various storage conditions.

| Storage conditions | Packing conditions | XRD | % Moisture Content Initial | % Moisture Content after storage |
|---|---|---|---|---|
| 40°C/75% relative humidity for 7 days | packed in double polythene bags and further kept in a fiber drum | Amorphous form conserved | 5.48% | 18.27% |
| 80°C for 7 days | open petriplate | Amorphous form conserved | 5.48% | 6.24% |
| Room temperature for 24 hours | open petriplate | Amorphous form conserved | 5.48% | 20.03% |
| Room temperature for 40 days | double polythene bags | Amorphous form conserved | 5.48% | 18.56 |

### Example 7

### HPLC Analysis method

**Reagent**: Water :milliQ,
Sodium perchlorate :AR Grade
Perchloric acid :AR Grade
Acetonitrile :J.T.Baker gradient
Trifluroacetic acid :AR Grade
**Buffer solution:** 6.1 g of sodium perchlorate into a 1000ml water. Adjust the pH to 3.0 (± 0.1) with perchloric acid.
**Mobile phase A:**
mixture of buffer and acetonitrile in the proportion of (90:10).
**Mobile phase B:**
mixture of buffer and acetonitrile in the proportion of (10 : 90).
**Diluent -1 :** mixture of water and acetonitrile in the ratio of 50 : 50.
**Diluent -2:** mixture of water and acetonitrile in the ratio of 90 : 10.
**Standard Stock Solution:**
Transfer accurately weighed 1.5 mg impurity-E RS and into a 200 ml volumetric flask. Dissolve in and dilute upto mark with diluent-1.

### Blank solution

Add 10 ml diluent-2 and 50µl of 3% trifluro acetic acid to a 50 ml volumetric flask, and dilute upto mark with diluent-2.

### System suitability solution

Transfer about 25 mg accurately weighed pemetrexed disodium sample in to a 50 ml volumetric flask. First add 10ml of diluent-2 and sonicate to dissolve the contents.Then add 50µl of 3% trifluro acetic acid (prepared in water) and add 5 ml of standard stock solution and dilute up to mark with diluent-2.

### Sample preparation

Transfer about 25 mg accurately weighed pemetrexed disodium sample in to a 50 ml volumetric flask. First add 10ml of diluent-2 and sonicate to dissolve the contents.Then add 50µl of 3% trifluro acetic acid (prepared in water) and dilute up to mark with diluent-2 (500 µg/ml).

### Chromatographic system :

Use a suitable high pressure liquid chromatography system equipped with Column: 250 mm x 4.6mm containing 5µ packing material (suggested column - Inertsil ODS 3V)
Detector: UV detector set to 240 nm
Cooler temp: 5°C.
Flow rate: about 1.5 ml/min.

The system is also equipped to deliver the two phases in a programmed manner as shown in the following table :

### Gradient programme :

| | | |
|---|---|---|
| 0 | 92 | 8 |
| 15 | 85 | 15 |
| 30 | 65 | 35 |
| 35 | 65 | 35 |
| 36 | 92 | 8 |
| 40 | 92 | 8 |

### Procedure:

Inject 20µl of blank and system suitability solution into the chromatograph set to above conditions and record the chromatograms up to 40 min.
Calculate the resolution between pemetrexed disodium and impurity-E. The resolution should not be less than 3.0. Calculate the Number of theoretical plate and tailing factor for pemetrexed peak. Number of theoretical plate is NLT 4000 and tailing factor is NMT 2.0.

Inject 20µl of test solution and calculate the chromatographic purity by area normalisation method.

## Claims

1. A stable amorphous form of pemetrexed disodium.

2. Amorphous pemetrexed disodium **characterized by** peaks in Raman spectrum at about (± 2cm⁻¹) 3064, 2921, 1611, 1534, 1438, 1343, 1293, 1190, 1157, 1076, 1010, 903, 872, 820, 639, 533, 373, 103 cm⁻¹.

3. A process for preparing an amorphous form of pemetrexed disodium comprising
a. dissolving pemetrexed disodium in a solvent to obtain a solution;
b. optionally filtering the solution ; and
c. recovering the amorphous form of pemetrexed disodium from the solution.

4. A process as claimed in claim 3, wherein the amorphous form of pemetrexed disodium is recovered from the solution by freeze drying, spray drying or flash drying.

5. A process as claimed in claim 3, wherein the solvent is selected from the group consisting of water and water and alkanol mixture.

6. A pharmaceutical composition comprising the amorphous form of pemetrexed disodium as claimed in claim 1 and pharmaceutically acceptable excipients.
